# EUROPÄISCHE PATENTSCHRIFT

(11) **EP 3 401 344 B1**
(45) Veröffentlichungstag und Bekanntmachung des Hinweises auf die Patenterteilung: **08.04.2020**
(21) Anmeldenummer: 17170116.2
(22) Anmeldetag: 09.05.2017
(51) Int. Cl.: C08G 18/75, C08G 18/02, C08G 18/09

(54) **VERFAHREN ZUR HERSTELLUNG VON TRIMEREN UND/ODER OLIGOMEREN VON DIISOCYANATEN**
METHOD FOR THE PREPARATION OF TRIMERS AND/OR OLIGOMERS OF DIISOCYANATES
PROCÉDÉ DE FABRICATION DE TRIMÈRES ET/OU D'OLIGOMÈRES DE DIISOCYANATES

(43) Veröffentlichungstag der Anmeldung: 14.11.2018
(73) Patentinhaber: Evonik Operations GmbH, 45128 Essen (DE)
(72) Erfinder: LOMÖLDER, Rainer, 48153 Münster (DE); NACKE, Christoph, 46514 Schermbeck (DE); SAJITZ, Melanie, 200030 Shanghai (CN); SPYROU, Emmanouil, 46514 Schermbeck (DE); HOPPE, Dirk, 48301 Nottuln (DE)
(74) Vertreter: Evonik Patent Association

(56) Entgegenhaltungen:
- CN-A- 103 450 443

## Beschreibung

Polyurethane sind wertvolle Rohstoffe für die Lack-, Kleb-, Dichtstoff- und Kunststoffindustrie. Meist werden sie erzeugt aus der Reaktion von Polyisocyanaten und Alkoholkomponenten. Um eine ausreichende Beständigkeit zu gewährleisten, ist es von Vorteil Polyisocyanate einzusetzen, die eine Funktionalität von >2 aufweisen. Besonders häufig werden zu diesem Zweck Isocyanurate verwendet, die aus der Trimerisierung und Oligomerisierung von Diisocyanaten resultieren. Zusätzlich wird durch eine solche Reaktion der Anteil an leicht flüchtigen und damit toxikologisch bedenklichen Diisocyanaten herabgesetzt. Bislang war aber nach einem solchen Trimerisierungsschritt eine aufwendige destillative Abtrennung des überschüssigen monomeren Diisocyanates notwendig.

Gemischte Trimerisierungen sind bereits beschrieben worden. So wurden in der DE 1954093 HDI und TDI gemeinsam trimerisiert und die überschüssigen Monomeren mit Hilfe einer Kurzweg- bzw. Dünnschichtdestillation abgetrennt. Dieses Verfahren konnte in EP0696606 dahingehend verbessert werden, dass durch hohe Gehalte an aliphatischen NCO-Gruppen der Anteil an aromatischen Restmonomeren auch ohne Destillation unter 0,5 Gew.-% herabgesetzt werden konnte. Beiden ist aber gemein, dass zum einen nicht UV-stabile aromatische Isocyanate zum Einsatz kommen, zum anderen aber große Anteile von monomeren Isocyanaten mit niedrigem Siedepunkt im Endprodukt verbleiben. Ganz ähnlich sieht es auch in dem chinesischen Patent CN 103450443 A (Preparation method of aromatic and aliphatic mixed isocyanurate curing agent) aus. In der DE19642324 kommt es zu Mischtrimerisierung von Cyclohexyldiisocyanat und aromatischen Diisocyanaten. Eine destillative Abtrennung von Monomeren ist hier nicht notwendig, aber auch hier verbleiben nicht-Licht-stabile aromatische Komponenten in dem Endprodukt.

In der US258482 werden Mischungen aus HDI und H12MDI trimerisiert nachdem zuvor ein Monoalkohol zugesetzt wurde, da dieser als notwendiger Cokatalysator fungiert. Nach der Trimerisierung muss der Trimerisierungskatalysator mit einem Inhibitor deaktiviert werden. Die entsprechenden Endprodukte weisen nach destillativer Abtrennung der monomeren Diisocyanate geringere Viskositäten auf, als entsprechende Vergleichsprodukte. Sowohl die Verwendung von Alkoholen (verringern Funktionalität und NCO-Gehalt) als auch die Notwendigkeit Inhibitoren zuzusetzen schränken die technische Verwendbarkeit dieser Lehre ein.

In der DE19627825 werden Mischungen aus Cyclohexyldiisocyanaten mit anderen Diisocyanaten (z.B. HDI, IPDI) mischtrimerisiert. Nachfolgend wird der Restmonomergehalt destillativ abgetrennt. In "Synthesis of HDI/IPDI hybrid isocyanurate and its application in polyurethane coating", Progress in Organic Coatings 78 (2015) 225-233, werden HDI und IPDI mischtrimerisiert, um in der Anwendung Vorteile, wie z.B. höheren TG oder aber bessere DOI, zu erzielen.

In der Vergangenheit hat es nicht an Bemühungen gemangelt, den Anteil an niedrig siedenden monomeren Diisocyanaten in einem Rohstoff oder einer Formulierung herabzusenken.

Üblicherweise geschieht dies mit Hilfe von destillativen Methoden, dies bedeutet aber sowohl einen zusätzlichen apparativen Aufwand, sowie eine Temperaturbelastung, die wiederum zu einer Veränderung, z.B. einer Verfärbung des Produktes führen kann.

Isocyanate zeigen bereits bei niedrigen Konzentrationen eine akute und chronische Wirkung bei Menschen. Daher liegen die Arbeitsplatzgrenzwerte für Diisocyanate nach TRGS 900 bei 0,02 bis 0,05 mg/m³. Neben der stoffspezifischen toxikologischen Wirkung unterscheiden sich Diisocyanate aber auch in ihrem Dampfdruck bzw. Siedepunkt. So werden bei gleichen Ausgangsvoraussetzungen je nach Dampfdruck bzw. Siedepunkt unterschiedlich große Diisocyanatmengen an die Raumluft abgeben, d.h. der zulässige Grenzwert wird früher, später oder nie erreicht. Insofern erscheint es im Sinne der Arbeitsplatzhygiene vorteilhaft, Diisocyanate so weit wie möglich zu vermeiden, die einen hohen Dampfdruck, bzw. einen niedrigen Siedepunkt aufweisen. Im Folgenden wird lediglich auf den Siedepunkt abgehoben, da dieser zum einen eng mit dem Dampfdruck zusammenhängt, zum anderen aber wesentlich zuverlässiger aus Literatur, bzw. Berechnungen zu entnehmen ist.

Zu einer einfacheren Betrachtung sollen die Diisocyanate abhängig von ihrem Siedepunkt in drei Kategorien eingeteilt werden, da mit einem höheren Siedepunkt die Flüchtigkeit und damit die Gefährlichkeit sinkt. Diisocyanate mit einem Siedepunkt von < 250 °C sollen als leicht flüchtig bezeichnet werden, Diisocyanate, mit einem Siedepunkt von 250-350 °C sollen als mittelflüchtige Diisocyanaten bezeichnet, und Diisocyanate, mit einem Siedepunkt von oberhalb von 350 °C, als schwer flüchtig bezeichnet werden.

Versucht man Isocyanurate mit einen niedrigen Monomergehalt an Diisocyanaten durch eine möglichst vollständige Trimerisierung/Oligomerisierung herzustellen, so beobachtet man zwei unerwünschte Effekte. Zum einen nimmt mit dem Fortschritt der Reaktion (Umsetzungsgrad), die Viskosität drastisch zu (Zunahme des Oligomerisierungsgrades bzw. der mittleren Molmasse). Zum anderen nimmt die Reaktion bei einer Minimalkonzentration an NCO-Gruppen immer stärker ab, so dass auch in Lösemitteln kein weiterer Umsatz erreicht werden kann. Daher verbleibt ein unerwünschter Teil an monomeren Diisocyanat im Endprodukt nach der Umsetzung. Diese Monomere müssen aus dem Endprodukt durch Destillation entfernt werden.

Aufgabe der vorliegenden Erfindung war es daher, Isocyanurate aus leicht und/oder mittel flüchtigen Diisocyanaten zugänglich zu machen, die einen niedrigen Monomergehalt an leicht und/oder mittel flüchtigen Diisocyanaten aufweisen, ohne aber auf den aufwendigen und belastenden Schritt der destillativen Abtrennung zurückgreifen zu müssen.

Überraschend wurde gefunden, dass Diisocyanate mit einem Siedepunkt von oberhalb von 350 °C, wie z.B. H12MDI, als Lösemittel verwendet werden können, um darin leicht und/oder mittelflüchtige Diisocyanate so zu trimerisieren, so dass auf die destillative Abtrennung der monomeren leicht und/oder mittelflüchtige Diisocyanate verzichtet werden kann.

Gegenstand der Erfindung ist eine Zusammensetzung von Trimeren und/oder Oligomeren aus Diisocyanaten und monomeren Diisocyanaten,
erhältlich durch Umsetzung von
I. 5-94,999 Gew.-%
   A) mindestens einem aliphatischen und/oder cycloaliphatischen Diisocyanat mit einem Siedepunkt von kleiner 250 °C
      und/oder
   B) mindestens einem aliphatischen und/oder cycloaliphatischen Diisocyanat mit einem Siedepunkt von 250-350 °C,
   in Anwesenheit von
II. 94,999-5 Gew.-%
   C) mindestens einem Diisocyanat mit einem Siedepunkt von oberhalb von 350 °C ausgewählt aus Dicyclohexylmethylendiisocyanat (H12MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-% relativ, beträgt.

Das bedeutet, mindestens 60 Gew.-% der eingesetzten Komponenten A) und/oder B) sind in Trimere und/oder Oligomere umgesetzt worden, bezogen auf die Summe von A) und B).

Gegenstand der Erfindung ist ein Verfahren zur Herstellung von Zusammensetzungen von Trimeren und/oder Oligomeren aus Diisocyanaten und monomeren Diisocyanaten,
erhältlich durch Umsetzung von
I. 5-94,999 Gew.-%
   A) mindestens einem aliphatischen und/oder cycloaliphatischen Diisocyanat mit einem Siedepunkt von kleiner 250 °C
      und/oder
   B) mindestens einem aliphatischen und/oder cycloaliphatischen Diisocyanat mit einem Siedepunkt von 250-350 °C,
   in Anwesenheit von
II. 94,999-5 Gew.-%
   C) mindestens einem Diisocyanat mit einem Siedepunkt von oberhalb von 350 °C ausgewählt aus Dicyclohexylmethylendiisocyanat (H12MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-% relativ, beträgt.

Das bedeutet, dass mindestens 60 Gew.-% der eingesetzten Komponenten A) und/oder B) in Trimere und/oder Oligomere umgesetzt worden sind, bezogen auf die Summe von A) und B).

Gegenstand der Erfindung ist auch die Verwendung
der Zusammensetzung von Trimeren und/oder Oligomeren aus Diisocyanaten und monomeren Diisocyanaten,
erhältlich durch Umsetzung von
I. 5-94,999 Gew.-%
   A) mindestens einem aliphatischen und/oder cycloaliphatischen Diisocyanat mit einem Siedepunkt von kleiner 250 °C
      und/oder
   B) mindestens einem aliphatischen und/oder cycloaliphatischen Diisocyanat mit einem Siedepunkt von 250-350 °C,
   in Anwesenheit von
II. 94,999-5 Gew.-%
   C) mindestens einem Diisocyanat mit einem Siedepunkt von oberhalb von 350 °C ausgewählt aus Dicyclohexylmethylendiisocyanat (H12MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte leicht- oder mittelflüchtige Diisocyanat A) bzw. B) nicht größer als 40 Gew.-% relativ, beträgt,
in Lacken, Klebstoffen, Dichtstoffen und/oder Kunststoffen.

Die Trimerisierung von Diisocyanaten ist bekannt und wurde schon häufig beschrieben. Grundsätzlich werden Polyisocyanurate durch katalytische Trimerisierung geeigneter Diisocyanate erhalten. Bei der Trimerisierung gemäß der Erfindung werden reine Trimere und/oder oligomere Trimere erhalten oder Mischtrimere bzw. Mischoligomere.

Geeignete Diisocyanate A) und B) im Sinn dieser Erfindung sind leicht flüchtige aliphatische, und/oder cycloaliphatische Diisocyanate A) und/oder mittel flüchtige aliphatische, und/oder cycloaliphatische Diisocyanate B).

Grundsätzlich können zur Trimerisierung geeignete Diisocyanate nach verschiedenartigen Verfahren hergestellt werden (Annalen der Chemie 562 (1949), S. 75ff.). Technisch insbesondere bewährt hat sich die Herstellung durch Phosgenierung von organischen Polyaminen zu den entsprechenden Polycarbaminsäurechloriden und deren thermische Spaltung in organische Polyisocyanate und Chlorwasserstoff. Alternativ können organische Polyisocyanate auch ohne die Verwendung von Phosgen, d. h. nach phosgenfreien Verfahren, hergestellt werden. Nach Angaben der EP-A-126 299 (USP 4,596,678), EP-A-126 300 (USP 4,596,679) und EP-A-355 443 (USP 5,087,739) beispielsweise können (cyclo)aliphatische Diisocyanate wie 1-Isocyanato-3-isocyanatomethyl-3,5,5-trimethylcyclohexan (Isophorondiisocyanat bzw. IPDI) zugänglich gemacht werden durch Umsetzung der zugrundeliegenden (cyclo)aliphatischen Diamine mit Harnstoff und Alkoholen zu (cyclo)aliphatischen Biscarbaminsäureestern und deren thermische Spaltung in die entsprechenden Diisocyanate und Alkohole.

Alle hier aufgeführten Siedepunkte zur Definition der Flüchtigkeit (bei Normaldruck (ND) = 1013 mbar) sind entweder aus der Literatur entnommen, oder aber aus Literaturdaten aus den Siedepunkten bei geringeren Drücken umgerechnet worden. Dazu wurde das folgende Umrechnungsprogramm von Sigma-Aldrich aus dem Internet verwendet: http://www.sigmaaldrich.com/chemistry/solvents/learning-center/nomo-assets.html.

Falls keine Literaturdaten bekannt waren, wurde der Siedepunkt mit Hilfe von Advanced Chem. Develop. Software V11.02, aus 2016, abgeschätzt.

Leicht flüchtige Diisocyanate A) sind z.B. Butyldiisocyanat Sdp.: 228 °C (ND), umgerechnet aus 112-113 °C bei 19 Torr (J. Polym. Sci., 1964, V2(8; Pt. A), P3387-404) und Ethyldiisocyanat, Sdp.: 189 °C (ND), umgerechnet aus 81 °C bei 20 Torr (Journal of Polymer Science 1964, V2(8; Pt. A), P3387 -404).

Mittel flüchtige Diisocyanate B) sind z.B. Isophorondiisocyanat (IPDI), Sdp.: 295 °C (ND), umgerechnet aus 217 °C bei 100 Torr (National Institut for Occupational Safety and Health), Hexamethylendiisocyanat (HDI), Sdp.: 255 °C (ND, Prog. in org. Coatings, 2010, V69(4), P426-341), Norbonandiisocyanat, Sdp.: 313 ± 15 °C (ND, berechnet mit Advanced Chem. Develop. Software V11.02), Mischung aus 2,2,4- und 2,4,4-Trimethylhexamethylendiisocyanat (TMDI), Sdp.: 284 °C (ND, berechnet mit Advanced Chem. Develop. Software V11.02) und Lysindiisocyanatethylester, Sdp.: 305 ± 37 °C (ND, berechnet mit Advanced Chem. Develop. Software V11.02).

Bevorzugt als mittelflüchtiges Diisocyanat B) wird IPDI.

Schwer flüchtige Diisocyanate C) sind Dicyclohexylmethylendiisocyanat (H₁₂MDI), Sdp.: 410 °C (ND), umgerechnet aus 156-158 °C bei 0,1 Torr aus Annalen der Chemie, 1949, 562, P75-136 und Octadekandiisocyanat, Sdp.: 435±18 °C (ND, berechnet mit Advanced Chem. Develop. Software V11.02).

Bevorzugt als schwer flüchtiges Diisocyanat C) wird H₁₂MDI.

Bevorzugter Gegenstand der Erfindung ist eine Zusammensetzung von Trimeren und/oder Oligomeren aus Diisocyanaten und monomeren Diisocyanaten,
erhältlich durch Umsetzung von
I. 5-94,999 Gew.-%
   A) mindestens einem aliphatischen und/oder cycloaliphatischen Diisocyanat mit einem Siedepunkt von kleiner 250 °C, ausgewählt aus Butyldiisocyanat ,Ethyldiisocyanat,
      und/oder
   B) mindestens einem aliphatischen und/oder cycloaliphatischen Diisocyanat mit einem Siedepunkt von 250-350 °C, ausgewählt aus Isophorondiisocyanat (IPDI), Hexamethylendiisocyanat (HDI), Norbonandiisocyanat, Mischung aus 2,2,4- und 2,4,4-Trimethylhexamethylendiisocyanat (TMDI) und Lysindiisocyanatethylester,
   in Anwesenheit von
II. 94,999-5 Gew.-%
   C) mindestens einem Diisocyanat mit einem Siedepunkt von oberhalb von 350 °C, ausgewählt aus Dicyclohexylmethylendiisocyanat (H₁₂MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-%relativ, beträgt.

Ganz besonders bevorzugter Gegenstand der Erfindung ist eine Zusammensetzung von Trimeren und/oder Oligomeren aus Diisocyanaten und monomeren Diisocyanaten,
erhältlich durch Umsetzung von
I. 5-94,999 Gew.-%
   B) Isophorondiisocyanat (IPDI)
   in Anwesenheit von
II. 94,999-5 Gew.-%
   C) Dicyclohexylmethylendiisocyanat (H12MDI),
III. in Gegenwart mindestens eines Trimerisierungs-Katalysators in Mengen von 0,001 bis 5 Gew.%, und die Mengen von I.-III. sich zu 100 Gew.-% addieren,
wobei der Anteil an monomeren B) nach der Umsetzung in der Mischung, bezogen auf die Gesamtmischung B)+C) absolut nicht größer als 20 Gew.-%, und bezogen auf das eingesetzte Diisocyanat B) nicht größer als 40 Gew.-% relativ, beträgt.

Als Trimerisierungs-Katalysatoren sind geeignet z. B. tert. Amine (US 3,996,223), Alkalimetallsalze von Carbonsäuren (CA 2113890; EP 56159), quartäre Ammoniumsalze (EP 798299; EP 524501; US 4,186,255; US 5,258,482; US 4,503,226; US 5,221,743), Aminosilane (EP 197864; US 4,697,014) und quartäre Hydroxyalkylammoniumsalze (EP 17998; US 4,324,879) und/oder quartäre Phosphoniumsalze. In Abhängigkeit vom Katalysator ist auch die Verwendung von diversen Co-Katalysatoren möglich, z. B. OH-funktionalisierte Verbindungen oder Mannich Basen aus sek. Aminen und Aldehyden bzw. Ketonen.

Zur Umsetzung läßt man die Diisocyanate in Gegenwart des Katalysators, ggf. unter Verwendung von Lösemitteln und/oder Hilfsstoffen, bis zum Erreichen des gewünschten Umsatzes reagieren. Danach kann die Reaktion durch Deaktivierung des Katalysators abgebrochen werden. Dies erfolgt durch Zusatz eines Katalysatorinhibitors. Besonders bevorzugt im Hinblick auf die Trimerisierung von Diisocyanaten im technischen Maßstab ist der Einsatz von quartären Hydroxyalkylammoniumcarboxylaten als Katalysatoren. Dieser Katalysatortyp ist thermisch labil und erlaubt eine gezielte thermische Deaktivierung, so dass es unnötig ist, die Trimerisierung bei Erreichen des gewünschten Umsatzes durch Zudosierung potentiell qualitätsmindernder Inhibitoren abzustoppen.

Als Katalysator werden bevorzugt quartäre Ammoniumsalze allein oder in Mischungen, besonders bevorzugt Tetralkylammoniumsalze und/oder quartäre Phosphoniumsalze, mit Halogenen, Hydroxiden, Alkoholaten oder organischen oder anorganischen Säureanionen als Gegenion, eingesetzt. Beispiele dafür sind:
Tetramethylammoniumformiat, Tetramethylammoniumacetat, Tetramethylammoniumpropionat, Tetramethylammoniumbutyrat, Tetramethylammonium-benzoat, Tetraethylammoniumformiat, Tetraethylammoniumacetat, Tetraethylammoniumpropionat, Tetraethylammoniumbutyrat, Tetraethylammoniumbenzoat, Tetrapropylammoniumformiat, Tetrapropylammoniumacetat, Tetrapropylammoniumpropionat, Tetrapropylammoniumbutyrat, Tetrapropylammoniumbenzoat, Tetrabutylammoniumformiat, Tetrabutylammoniumacetat, Tetrabutylammoniumpropionat, Tetrabutylammoniumbutyrat und Tetrabutylammoniumbenzoat und Tetrabutylphosphoniumacetat, Tetrabutylphosphoniumformiat und Ethyltriphenylphosphoniumacetat, Tetrabutylphosphoniumbenzotriazolat, Tetraphenylphosphoniumphenolat und Trihexyltetradecylphosphoniumdecanoat, Methyltributylammoniumhydroxid, Methyltriethylammoniumhydroxid, Tetramethylammoniumhydroxid, Tetraethylammoniumhydroxid, Tetrapropylammoniumhydroxid, Tetrabutylammoniumhydroxid, Tetrapentylammoniumhydroxid, Tetrahexylammoniumhydroxid, Tetraoctylammoniumhydroxid, Tetradecylammoniumhydroxid, Tetradecyltrihexylammoniumhydroxid, Tetraoctadecylammoniumhydroxid, Benzyltrimethylammoniumhydroxid, Benzyltriethylammoniumhydroxid, Trimethylphenylammoniumhydroxid, Triethylmethylammoniumhydroxid, Trimethylvinylammoniumhydroxid, Methyltributylammoniummethanolat, Methyltriethylammoniummethanolat, Tetramethylammoniummethanolat, Tetraethylammoniummethanolat, Tetrapropylammoniummethanolat, Tetrabutylammoniummethanolat, Tetrapentylammoniummethanolat, Tetrahexylammoniummethanolat, Tetraoctylammoniummethanolat, Tetradecylammoniummethanolat, Tetradecyltrihexylammoniummethanolat, Tetraoctadecylammoniummethanolat, Benzyltrimethylammoniummethanolat, Benzyltriethylammoniummethanolat, Trimethylphenylammoniummethanolat, Triethylmethylammoniummethanolat, Trimethylvinylammoniummethanolat, Methyltributylammoniumethanolat, Methyltriethylammoniumethanolat, Tetramethylammoniumethanolat, Tetraethylammoniumethanolat, Tetrapropylammoniumethanolat, Tetrabutylammoniumethanolat, Tetrapentylammoniumethanolat, Tetrahexylammoniumethanolat, Tetraoctylammoniummethanolat, Tetradecylammoniumethanolat, Tetradecyltrihexylammoniumethanolat, Tetraoctadecylammoniumethanolat, Benzyltrimethylammoniumethanolat, Benzyltriethylammoniumethanolat, Tri methylphenylammoniumethanolat, Triethylmethylammoniumethanolat, Tri methylvinylammoniumethanolat, Methyltributylammoniumbenzylat, Methyltriethylammoniumbenzylat, Tetramethylammoniumbenzylat, Tetraethylammoniumbenzylat, Tetrapropylammoniumbenzylat, Tetrabutylammoniumbenzylat, Tetrapentylammoniumbenzylat, Tetrahexylammoniumbenzylat, Tetraoctylammoniumbenzylat, Tetradecylammoniumbenzylat, Tetradecyltrihexylammoniumbenzylat, Tetraoctadecylammoniumbenzylat, Benzyltrimethylammoniumbenzylat, Benzyltriethylammoniumbenzylat, Trimethylphenylammoniumbenzylat, Triethylmethylammoniumbenzylat, Trimethylvinylammoniumbenzylat, Tetramethylammoniumfluorid, Tetraethylammoniumfluorid, Tetrabutylammoniumfluorid, Tetraoctylammoniumfluorid, Benzyltrimethylammoniumfluorid, Tetrabutylphosphoniumhydroxid, Tetrabutylphosphoniumfluorid, Tetrabutylammoniumchlorid, Tetrabutylammoniumbromid, Tetrabutylammoniumiodid, Tetraethylammoniumchlorid, Tetraethylammoniumbromid, Tetraethylammoniumiodid, Tetramethylammoniumchlorid, Tetramethylammoniumbromid, Tetramethylammoniumiodid, Benzyltrimethylammoniumchlorid, Benzyltriethylammoniumchlorid, Benzyltripropylammoniumchlorid, Benzyltributylammoniumchlorid, Methyltributylammoniumchlorid, Methyltripropylammoniumchlorid, Methyltriethylammoniumchlorid, Methyltriphenylammoniumchlorid, Phenyltrimethylammoniumchlorid, Benzyltrimethylammoniumbromid, Benzyltriethylammoniumbromid, Benzyltripropylammoniumbromid, Benzyltributylammoniumbromid, Methyltributylammoniumbromid, Methyltripropylammoniumbromid, Methyltriethylammoniumbromid, Methyltriphenylammoniumbromid, Phenyltrimethylammoniumbromid, Benzyltrimethylammoniumiodid, Benzyltriethylammoniumiodid, Benzyltripropylammoniumiodid, Benzyltributylammoniumiodid, Methyltributylammoniumiodid, Methyltripropylammoniumiodid, Methyltriethylammoniumiodid, Methyltriphenylammoniumiodid und Phenyltrimethylammoniumiodid, Methyltributylammoniumhydroxid, Methyltriethylammoniumhydroxid, Tetramethylammoniumhydroxid, Tetraethylammoniumhydroxid, Tetrapropylammoniumhydroxid, Tetrabutylammoniumhydroxid, Tetrapentylammoniumhydroxid, Tetrahexylammoniumhydroxid, Tetraoctylammoniumhydroxid, Tetradecylammoniumhydroxid, Tetradecyltrihexylammoniumhydroxid, Tetraoctadecylammoniumhydroxid, Benzyltrimethylammoniumhydroxid, Benzyltriethylammoniumhydroxid, Trimethylphenylammoniumhydroxid, Triethylmethylammoniumhydroxid, Trimethylvinylammoniumhydroxid, Tetramethylammoniumfluorid, Tetraethylammoniumfluorid, Tetrabutylammoniumfluorid, Tetraoctylammoniumfluorid und Benzyltrimethylammoniumfluorid. Diese Katalysatoren können allein oder in Mischungen zugesetzt werden. Bevorzugt wird Tetraethylammoniumhydroxid verwendet.

Erfindungsgemäß wird die Trimerisierung entweder batchweise oder kontinuierlich durchgeführt. Das Batchverfahren wird bevorzugt. Beim Batchverfahren wird in einem Rührreaktor in der Regel bei Umgebungsdruck (Normaldruck 1013 mbar) gearbeitet, aber auch andere Drücke sind möglich. Dabei wird ein Gemisch aus 94,999-5 Gew.-% C) und 5-94,999 Gew.- % eines weiteren leichtflüchtigen Diisocyanats A) und/oder mittelflüchtigen Diisocyanats B) vorgelegt. Zunächst wird die Mischung der Diisocyanate auf eine Temperatur von 0 - 140°C, vorzugsweise von 55 - 90°C, besonders bevorzugt von 65 - 75°C vorgeheizt, Dann wird der Katalysator in Mengen von 0,001 bis 5 Gew.-% zudosiert, Die Trimerisierung ist exotherm. Der Katalysator wird zunächst in einer solchen Menge zudosiert, dass ein deutlicher Anstieg der Temperatur der Reaktionsmischung um 5 - 15°C erkennbar wird. Da der Katalysator im Zuge der Reaktion deaktiviert wird, sinkt die Temperatur der Reaktionsmischung im Verlauf der Umsetzung wieder ab und eine erneute Katalysatordosierung kann erfolgen. Dieser Vorgang wird wiederholt bis der gewünschte Umsatz erreicht ist. Die Abfolge von Katalysatordeaktivierung und Reinitiierung der Trimerisierung durch portionsweise Katalysatornachdosierung erlaubt dabei jederzeit eine optimale Prozesskontrolle sowohl in Bezug auf den Umsatz als auch hinsichtlich des Temperaturprofils der Reaktion.

Der Katalysator kann in reiner Form eingesetzt werden. Zur exakteren Dosierung und optimalen Durchmischung des Katalysators ist es jedoch vorteilhaft, den Katalysator in einem geeigneten Lösemittel zu lösen. Geeignet sind prinzipiell solche Lösemittel, in denen der Katalysator eine gute Löslichkeit hat, z. B. Wasser, niedermolekulare Alkohole wie Methanol oder Ethylenglykol oder niedermolekulare organische Säuren wie beispielsweise Essigsäure oder Hexansäure.

Die kontinuierliche Trimerisierung kann in einer Kesselkaskade durchgeführt werden. Denkbar ist auch eine Kombination aus Kesselkaskade und Rohrreaktor.

Zur Beschränkung der auf den gewünschten Umsatz bezogenen notwendigen Katalysatormenge sollte das Temperaturprofil des erfindungsgemäßen Verfahrens so eingerichtet werden, dass die Reaktionslösung eine Temperatur von 95 °C möglichst nicht überschreitet.
Nach der Trimerisierungsreaktion kann optional ein Katalysatorgift zugesetzt, meist ist dies jedoch nicht notwendig.

Das entstandene erfindungsgemäße Zusammensetzung enthält neben den Restmonomeren noch reine und gemischte Trimerisate und Oligomerisate der eingesetzten Diisocyanate A)und/oder B) und C). Diese Mischung kann so wie sie ist oder in Abmischungen als NCO-Komponente in Lack-Klebstoff, Dichtstoff oder Kunststoffformulierungen verwendet werden.

Die Trimerisierung wird soweit fortgesetzt, dass der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte leicht- oder mittelflüchtige Diisocyanat A) bzw. B) nicht größer als 40 Gew.-% relativ, beträgt.

Bevorzugt werden absolute Werte von <10 Gew.-% und relativ <20 Gew.-%. Besonders bevorzugt werden Werte von absolut <3 Gew.-% und relativ <6 Gew.-%.

### Beispiele

### Allgemeine Arbeitsvorschrift

Ein Gemisch aus 70 Gew.-% VESTANAT H₁₂MDI, C) Dicyclohexylmethylendiisocyanat, (Sdp.: 410 °C Evonik Industries AG) und 30 Gew.-% VESTANAT IPDI, B) Isophorondiisocyanat (IPDI), (Sdp.: 295 °C, Evonik Industries AG), wird in einem gerührten Dreihalskolben auf eine bestimmte Starttemperatur aufgeheizt und mit einem Katalysator (Tetraethylammoniumhydroxid (TEAOH, Aldrich) versetzt, worauf sofort eine exotherme Reaktion einsetzt. Nach Zugabe des Katalysators wird die Heizquelle entfernt und das Produkt kühlt sich nach wenigen Minuten der exothermen Reaktion langsam ab. Nach Abkühlung wird die dem Katalysator äquivalente Menge an p-Toluolsulfonsäure (Aldrich) zugesetzt. Dabei entsprechen 91 g Tetraethylammoniumhydroxid 172 g p-Toluolsulfonsäure.

| | **Katalysator** | **Start Temperatur** | **NCO Gehalt** | **Temp. Maximum** | **Freies IPDI (relativ zu Ausgang)** | **freies H₁₂MDI** |
|---|---|---|---|---|---|---|
| | TEAOH [Gew.-%] | [°C] | [Gew.-%]???? | [°C] | [Gew.-%] | [Gew.-%] |
| Versuch 1 | 0,25 | 80 | 24,2 | 131 | 3,7 (10,2) | 49,2 |
| Versuch 2 | 0,25 | 100 | 25,9 | 137 | 7,4 (24,7) | 54,4 |
| Versuch 3 | 0,5 | 80 | 21,2 | 142 | 0,5 (1,7) | 37,1 |
| Versuch 4 | 0,2 | 80 | 24,7 | 128 | 4,7 (15,7) | 51,3 |
| Versuch 5 | 0,1 | 80 | 27,3 | 120 | 10,4 (34,7) | 62,8 |

Der Restmonomergehalt an IPDI beträgt in allen Versuchen weniger als 20 Gew.-% (Freies IPDI, linke Spalte, absolut). Bezogen auf das eingesetzte IPDI ist ein relativer Restmonomergehalt von ≤40 Gew.-% zu beobachten (Freies IPDI, rechte Spalte in Klammern, relativ) berechnet aus: (Endkonzentration IPDI / Ausgangskonzentration IPDI) X 100 = relativer Restmonomergehalt IPDI

## Patentansprüche

1. Zusammensetzung von Trimeren und/oder Oligomeren aus Diisocyanaten und monomeren Diisocyanaten,
erhältlich durch Umsetzung von
I. 5-94,999 Gew.-%
A) mindestens einem aliphatischen und/oder cycloaliphatischen Diisocyanat mit einem Siedepunkt von kleiner 250 °C (bei Normaldruck)
und/oder
B) mindestens einem aliphatischen und/oder cycloaliphatischen Diisocyanat mit einem Siedepunkt von 250-350 °C (bei Normaldruck),
in Anwesenheit von
II. 94,999-5 Gew.-%
C) mindestens einem Diisocyanat mit einem Siedepunkt von oberhalb von 350 °C (bei Normaldruck) ausgewählt aus Dicyclohexylmethylendiisocyanat (H₁₂MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-% relativ, beträgt.

2. Zusammensetzung von Trimeren und/oder Oligomeren aus Diisocyanaten und monomeren Diisocyanaten nach Anspruch 1, wobei mindestens 60 Gew.-% der eingesetzten Komponenten A) und/oder B) in Trimere und/oder Oligomere umgesetzt worden sind, bezogen auf die Summe von A) und B).

3. Zusammensetzung von Trimeren und/oder Oligomeren aus Diisocyanaten und monomeren Diisocyanaten, nach mindestens einem der vorherigen Ansprüche, wobei mindestens ein Diisocyanat A) mit einem Siedepunkt von kleiner 250 °C, ausgewählt aus Butyldiisocyanat und Ethyldiisocyanat, eingesetzt wird.

4. Zusammensetzung von Trimeren und/oder Oligomeren aus Diisocyanaten und monomeren Diisocyanaten nach mindestens einem der vorherigen Ansprüche, wobei mindestens ein Diisocyanat B) mit einem Siedepunkt von 250-350 °C, ausgewählt aus Isophorondiisocyanat (IPDI), Hexamethylendiisocyanat (HDI), Norbonandiisocyanat, Mischung aus 2,2,4- und 2,4,4-Trimethylhexamethylendiisocyanat (TMDI), und/oder Lysindiisocyanatethylester, eingesetzt wird.

5. Zusammensetzung von Trimeren und/oder Oligomeren aus Diisocyanaten und monomeren Diisocyanaten nach mindestens einem der vorherigen Ansprüche, erhältlich durch Umsetzung von
I. 5-94,999 Gew.-%
A) mindestens einem Diisocyanat mit einem Siedepunkt von kleiner 250 °C, ausgewählt aus Butyldiisocyanat und Ethyldiisocyanat,
und/oder
B) mindestens einem Diisocyanat mit einem Siedepunkt von 250-350 °C, ausgewählt aus Isophorondiisocyanat (IPDI), Hexamethylendiisocyanat (HDI), Norbonandiisocyanat, Mischung aus 2,2,4- und 2,4,4-Trimethylhexamethylendiisocyanat (TMDI) und Lysindiisocyanatethylester,
in Anwesenheit von
II. 94,999-5 Gew.-%
C) mindestens einem Diisocyanat mit einem Siedepunkt von oberhalb von 350 °C, ausgewählt aus Dicyclohexylmethylendiisocyanat (H₁₂MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-% relativ, beträgt.

6. Zusammensetzung von Trimeren und/oder Oligomeren aus Diisocyanaten und monomeren Diisocyanaten nach mindestens einem der vorherigen Ansprüche, erhältlich durch Umsetzung von
I. 5-94,999 Gew.-%
B) Isophorondiisocyanat (IPDI)
in Anwesenheit von
II. 94,999-5 Gew.-%
C) Dicyclohexylmethylendiisocyanat (H12MDI),
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren B) nach der Umsetzung in der Mischung, bezogen auf die Gesamtmischung B)+C) absolut nicht größer als 20 Gew.-%, und bezogen auf das eingesetzte Diisocyanat B) nicht größer als 40 Gew.-% relativ, beträgt.

7. Zusammensetzung von Trimeren und/oder Oligomeren aus Diisocyanaten und monomeren Diisocyanaten nach mindestens einem der vorherigen Ansprüche, wobei als als Trimerisierungs-Katalysatoren III. tertiäre Amine, Alkalimetallsalze von Carbonsäuren, quartäre Ammoniumsalze, Aminosilane quartanäre Hydroxyalkylammoniumsalze, quartäre Phosphoniumsalze allein oder in Mischungen, eingesetzt werden.

8. Zusammensetzung von Trimeren und/oder Oligomeren aus Diisocyanaten und monomeren Diisocyanaten nach mindestens einem der vorherigen Ansprüche, wobei Trimerisierungs-Katalysatoren III ausgewählt aus quartären Ammoniumsalzen und quartären Phosphoniumsalzen, allein oder in Mischungen eingesetzt werden.

9. Zusammensetzung von Trimeren und/oder Oligomeren aus Diisocyanaten und monomeren Diisocyanaten nach mindestens einem der vorherigen Ansprüche, wobei Trimerisierungs-Katalysatoren III ausgewählt aus quartären Tetralkylammoniumsalzen und/oder quartären Phosphoniumsalzen, mit Halogenen, Hydroxiden, Alkoholaten oder organischen oder anorganischen Säureanionen als Gegenion, allein oder in Mischungen eingesetzt werden.

10. Zusammensetzung von Trimeren und/oder Oligomeren aus Diisocyanaten und monomeren Diisocyanaten nach mindestens einem der vorherigen Ansprüche, wobei als Trimerisierungs-Katalysator III. Tetraethylammoniumhydroxid eingesetzt wird.

11. Verfahren zur Herstellung von Zusammensetzungen von Trimeren und/oder Oligomeren aus Diisocyanaten und monomeren Diisocyanaten, nach mindestens einem der vorherigen Ansprüche,
erhältlich durch Umsetzung von
I. 5-94,999 Gew.-%
A) mindestens einem aliphatischen und/oder cycloaliphatischen Diisocyanat mit einem Siedepunkt von kleiner 250 °C
und/oder
B) mindestens einem aliphatischen und/oder cycloaliphatischen Diisocyanat mit einem Siedepunkt von 250-350 °C,
in Anwesenheit von
II. 94,999-5 Gew.-%
C) mindestens einem Diisocyanat mit einem Siedepunkt von oberhalb von 350 °C ausgewählt aus Dicyclohexylmethylendiisocyanat (H₁₂MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und, bezogen auf das eingesetzte Diisocyanat A) und/oder B) nicht größer als 40 Gew.-% relativ, beträgt.

12. Verfahren zur Herstellung von Zusammensetzungen von Trimeren und/oder Oligomeren aus Diisocyanaten und monomeren Diisocyanaten, nach Anspruch 11, wobei die Umsetzung bei Temperaturen von 0 - 140°C, vorzugsweise von 55 - 90°C, besonders bevorzugt von 65 - 75°C, erfolgt.

13. Verwendung der Zusammensetzung von Trimeren und/oder Oligomeren aus Diisocyanaten und monomeren Diisocyanaten nach mindestens einem der vorherigen Ansprüche,
erhältlich durch Umsetzung von
I. 5-94,999 Gew.-%
A) mindestens einem aliphatischen und/oder cycloaliphatischen Diisocyanat mit einem Siedepunkt von kleiner 250 °C
und/oder
B) mindestens einem aliphatischen und/oder cycloaliphatischen Diisocyanat mit einem Siedepunkt von 250-350 °C,
in Anwesenheit von
II. 94,999-5 Gew.-%
C) mindestens einem Diisocyanat mit einem Siedepunkt von oberhalb von 350 °C ausgewählt aus Dicyclohexylmethylendiisocyanat (H₁₂MDI) und/oder Octadekandiisocyanat,
III. in Gegenwart mindestens eines Trimerisierungs-Katalysators in Mengen von 0,001 bis 5 Gew.%,
und die Mengen von I.-III. sich zu 100 Gew.-% addieren,
wobei der Anteil an monomeren A) und/oder monomeren B) nach der Umsetzung in der Mischung, bezogen auf die Gesamtmischung A)+B)+C) absolut nicht größer als 20 Gew.-%, und bezogen auf das eingesetzte Diisocyanat A) und/oder B) nicht größer als 40 Gew.-% relativ, beträgt,
in Lacken, Klebstoffen, Dichtstoffen und/oder Kunststoffen.

## Claims

1. Composition of trimers and/or oligomers formed from diisocyanates and monomeric diisocyanates, obtainable by reaction of
I. 5-94.999% by weight of
A) at least one aliphatic and/or cycloaliphatic diisocyanate having a boiling point of less than 250°C (at standard pressure)
and/or
B) at least one aliphatic and/or cycloaliphatic diisocyanate having a boiling point of 250-350°C (at standard pressure),
in the presence of
II. 94.999-5% by weight of
C) at least one diisocyanate having a boiling point above 350°C (at standard pressure), selected from dicyclohexylmethylene diisocyanate (H₁₂MDI) and/or octadecane diisocyanate,
III. in the presence of at least one trimerization catalyst in amounts of 0.001% to 5% by weight,
and the amounts of I.-III. add up to 100% by weight,
where the proportion of monomeric A) and/or monomeric B) after the reaction in the mixture, based on the overall mixture of A)+B)+C), in absolute terms is not greater than 20% by weight, and based on the diisocyanate A) and/or B) used is not greater than 40% by weight in relative terms.

2. Composition of trimers and/or oligomers formed from diisocyanates and monomeric diisocyanates according to Claim 1, wherein at least 60% by weight of the components A) and/or B) used has been converted to trimers and/or oligomers, based on the sum total of A) and B).

3. Composition of trimers and/or oligomers formed from diisocyanates and monomeric diisocyanates according to at least one of the preceding claims, wherein at least one diisocyanate A) having a boiling point of less than 250°C, selected from butyl diisocyanate and ethyl diisocyanate, is used.

4. Composition of trimers and/or oligomers formed from diisocyanates and monomeric diisocyanates according to at least one of the preceding claims, wherein at least one diisocyanate B) having a boiling point of 250-350°C, selected from isophorone diisocyanate (IPDI), hexamethylene diisocyanate (HDI), norbornane diisocyanate, mixture of 2,2,4- and 2,4,4-trimethylhexamethylene diisocyanate (TMDI) and/or lysine diisocyanate ethyl ester, is used.

5. Composition of trimers and/or oligomers formed from diisocyanates and monomeric diisocyanates according to at least one of the preceding claims, obtainable by reaction of
I. 5-94.999% by weight of
A) at least one diisocyanate having a boiling point of less than 250°C, selected from butyl diisocyanate and ethyl diisocyanate,
and/or
B) at least one diisocyanate having a boiling point of 250-350°C, selected from isophorone diisocyanate (IPDI), hexamethylene diisocyanate (HDI), norbornane diisocyanate, mixture of 2,2,4- and 2,4,4-trimethylhexamethylene diisocyanate (TMDI) and lysine diisocyanate ethyl ester,
in the presence of
II. 94.999-5% by weight of
C) at least one diisocyanate having a boiling point above 350°C, selected from dicyclohexylmethylene diisocyanate (H₁₂MDI) and/or octadecane diisocyanate,
III. in the presence of at least one trimerization catalyst in amounts of 0.001% to 5% by weight,
and the amounts of I.-III. add up to 100% by weight,
where the proportion of monomeric A) and/or monomeric B) after the reaction in the mixture, based on the overall mixture of A)+B)+C), in absolute terms is not greater than 20% by weight, and based on the diisocyanate A) and/or B) used is not greater than 40% by weight in relative terms.

6. Composition of trimers and/or oligomers formed from diisocyanates and monomeric diisocyanates according to at least one of the preceding claims, obtainable by reaction of
I. 5-94.999% by weight of
B) isophorone diisocyanate (IPDI)
in the presence of
II. 94.999-5% by weight of
C) dicyclohexylmethylene diisocyanate (H12MDI),
III. in the presence of at least one trimerization catalyst in amounts of 0.001% to 5% by weight,
and the amounts of I.-III. add up to 100% by weight,
where the proportion of monomeric B) after the reaction in the mixture, based on the overall mixture of B)+C), in absolute terms is not greater than 20% by weight, and based on the diisocyanate B) used is not greater than 40% by weight in relative terms.

7. Composition of trimers and/or oligomers formed from diisocyanates and monomeric diisocyanates according to at least one of the preceding claims, wherein the trimerization catalysts III. used are tertiary amines, alkali metal salts of carboxylic acids, quaternary ammonium salts, aminosilanes, quaternary hydroxyalkylammonium salts, quaternary phosphonium salts, on their own or in mixtures.

8. Composition of trimers and/or oligomers formed from diisocyanates and monomeric diisocyanates according to at least one of the preceding claims, wherein trimerization catalysts III. selected from quaternary ammonium salts and quaternary phosphonium salts are used, on their own or in mixtures.

9. Composition of trimers and/or oligomers formed from diisocyanates and monomeric diisocyanates according to at least one of the preceding claims, wherein trimerization catalysts III. selected from quaternary tetraalkylammonium salts and/or quaternary phosphonium salts, with halogens, hydroxides, alkoxides or organic or inorganic acid anions as counterion, are used, on their own or in mixtures.

10. Composition of trimers and/or oligomers formed from diisocyanates and monomeric diisocyanates according to at least one of the preceding claims, wherein the trimerization catalyst III. used is tetraethylammonium hydroxide.

11. Process for producing compositions of trimers and/or oligomers from diisocyanates and monomeric diisocyanates according to at least one of the preceding claims,
obtainable by reaction of
I. 5-94.999% by weight of
A) at least one aliphatic and/or cycloaliphatic diisocyanate having a boiling point of less than 250°C
and/or
B) at least one aliphatic and/or cycloaliphatic diisocyanate having a boiling point of 250-350°C,
in the presence of
II. 94.999-5% by weight of
C) at least one diisocyanate having a boiling point above 350°C selected from dicyclohexylmethylene diisocyanate (H₁₂MDI) and/or octadecane diisocyanate,
III. in the presence of at least one trimerization catalyst in amounts of 0.001% to 5% by weight,
and the amounts of I.-III. add up to 100% by weight,
where the proportion of monomeric A) and/or monomeric B) after the reaction in the mixture, based on the overall mixture of A)+B)+C), in absolute terms is not greater than 20% by weight, and based on the diisocyanate A) and/or B) used is not greater than 40% by weight in relative terms.

12. Process for producing compositions of trimers and/or oligomers from diisocyanates and monomeric diisocyanates according to Claim 11, wherein the reaction is effected at temperatures of 0-140°C, preferably of 55-90°C, more preferably of 65-75°C.

13. Use of the composition of trimers and/or oligomers formed from diisocyanates and monomeric diisocyanates according to at least one of the preceding claims,
obtainable by reaction of
I. 5-94.999% by weight of
A) at least one aliphatic and/or cycloaliphatic diisocyanate having a boiling point of less than 250°C
and/or
B) at least one aliphatic and/or cycloaliphatic diisocyanate having a boiling point of 250-350°C,
in the presence of
II. 94.999-5% by weight of
C) at least one diisocyanate having a boiling point above 350°C selected from dicyclohexylmethylene diisocyanate (H₁₂MDI) and/or octadecane diisocyanate,
III. in the presence of at least one trimerization catalyst in amounts of 0.001% to 5% by weight,
and the amounts of I.-III. add up to 100% by weight,
where the proportion of monomeric A) and/or monomeric B) after the reaction in the mixture, based on the overall mixture of A)+B)+C), in absolute terms is not greater than 20% by weight, and based on the diisocyanate A) and/or B) used is not greater than 40% by weight in relative terms,
in paints, adhesives, sealants and/or plastics.

## Revendications

1. Composition de trimères et/ou d'oligomères de diisocyanates et de diisocyanates monomères,
pouvant être obtenue par mise en réaction de
I. 5 à 94,999 % en poids de
A) au moins un diisocyanate aliphatique et/ou cycloaliphatique ayant un point d'ébullition inférieur à 250 °C (à pression normale),
et/ou
B) au moins un diisocyanate aliphatique et/ou cycloaliphatique ayant un point d'ébullition de 250 à 350 °C (à pression normale), en présence de
II. 94,999 à 5 % en poids de
C) au moins un diisocyanate ayant un point d'ébullition supérieur à 350 °C (à pression normale) choisi parmi le diisocyanate de dicyclohexylméthylène (H₁₂MDI) et/ou le diisocyanate d'octadécane,
III. en présence d'au moins un catalyseur de trimérisation en quantités de 0,001 à 5 % en poids,
la somme des quantités de I. à III. étant de 100 % en poids,
la proportion de A) monomère et/ou de B) monomère après la réaction dans le mélange, par rapport au mélange total A)+B)+C), n'étant absolument pas supérieure à 20 % en poids et, par rapport au diisocyanate A) et/ou B) utilisé, n'étant relativement pas supérieure à 40 % en poids.

2. Composition de trimères et/ou d'oligomères de diisocyanates et de diisocyanates monomères selon la revendication 1, dans laquelle au moins 60 % en poids des composants A) et/ou B) utilisés ont été transformés en trimères et/ou oligomères, par rapport à la somme de A) et B).

3. Composition de trimères et/ou d'oligomères de diisocyanates et de diisocyanates monomères selon au moins l'une quelconque des revendications précédentes, dans laquelle au moins un diisocyanate A) ayant un point d'ébullition inférieur à 250 °C, choisi parmi le diisocyanate de butyle et le diisocyanate d'éthyle, est utilisé.

4. Composition de trimères et/ou d'oligomères de diisocyanates et de diisocyanates monomères selon au moins l'une quelconque des revendications précédentes, dans laquelle au moins un diisocyanate B) ayant un point d'ébullition de 250 à 350 °C, choisi parmi le diisocyanate d'isophorone (IPDI), le diisocyanate d'hexaméthylène (HDI), le diisocyanate de norbornane, un mélange de diisocyanate de 2,2,4- et 2,4,4-triméthylhexaméthylène (TMDI) et/ou l'ester éthylique de diisocyanate de lysine, est utilisé.

5. Composition de trimères et/ou d'oligomères de diisocyanates et de diisocyanates monomères selon au moins l'une quelconque des revendications précédentes, pouvant être obtenue par mise en réaction de
I. 5 à 94,999 % en poids de
A) au moins un diisocyanate ayant un point d'ébullition inférieur à 250 °C, choisi parmi le diisocyanate de butyle et le diisocyanate d'éthyle, et/ou
B) au moins un diisocyanate ayant un point d'ébullition de 250 à 350 °C, choisi parmi le diisocyanate d'isophorone (IPDI), le diisocyanate d'hexaméthylène (HDI), le diisocyanate de norbornane, un mélange de diisocyanate de 2,2,4- et 2,4,4-triméthylhexaméthylène (TMDI) et l'ester éthylique de diisocyanate de lysine,
en présence de
II. 94,999 à 5 % en poids de
C) au moins un diisocyanate ayant un point d'ébullition supérieur à 350 °C, choisi parmi le diisocyanate de dicyclohexylméthylène (H₁₂MDI) et/ou le diisocyanate d'octadécane,
III. en présence d'au moins un catalyseur de trimérisation en quantités de 0,001 à 5 % en poids,
la somme des quantités de I. à III. étant de 100 % en poids,
la proportion de A) monomère et/ou de B) monomère après la réaction dans le mélange, par rapport au mélange total A)+B)+C), n'étant absolument pas supérieure à 20 % en poids et, par rapport au diisocyanate A) et/ou B) utilisé, n'étant relativement pas supérieure à 40 % en poids.

6. Composition de trimères et/ou d'oligomères de diisocyanates et de diisocyanates monomères selon au moins l'une quelconque des revendications précédentes, pouvant être obtenue par mise en réaction de
I. 5 à 94,999 % en poids de
B) diisocyanate d'isophorone (IPDI),
en présence de
II. 94,999 à 5 % en poids de
C) diisocyanate de dicyclohexylméthylène (H₁₂MDI),
III. en présence d'au moins un catalyseur de trimérisation en quantités de 0,001 à 5 % en poids,
la somme des quantités de I. à III. étant de 100 % en poids,
la proportion de B) monomère après la réaction dans le mélange, par rapport au mélange total B)+C), n'étant absolument pas supérieure à 20 % en poids et, par rapport au diisocyanate B) utilisé, n'étant relativement pas supérieure à 40 % en poids.

7. Composition de trimères et/ou d'oligomères de diisocyanates et de diisocyanates monomères selon au moins l'une quelconque des revendications précédentes, dans laquelle des amines tertiaires, des sels de métaux alcalins d'acides carboxyliques, des sels d'ammonium quaternaires, des aminosilanes, des sels d'hydroxyalkylammonium quaternaires, des sels de phosphonium quaternaires seuls ou en mélanges sont utilisés en tant que catalyseurs de trimérisation III.

8. Composition de trimères et/ou d'oligomères de diisocyanates et de diisocyanates monomères selon au moins l'une quelconque des revendications précédentes, dans laquelle des catalyseurs de trimérisation III choisis parmi les sels d'ammonium quaternaires et les sels de phosphonium quaternaires, seuls ou en mélanges, sont utilisés.

9. Composition de trimères et/ou d'oligomères de diisocyanates et de diisocyanates monomères selon au moins l'une quelconque des revendications précédentes, dans laquelle des catalyseurs de trimérisation III choisis parmi les sels de tétraalkylammonium quaternaires et/ou les sels de phosphonium quaternaires, avec des halogènes, des hydroxydes, des alcoolates ou des anions d'acides organiques ou inorganiques en tant que contre-ion, seuls ou en mélanges, sont utilisés.

10. Composition de trimères et/ou d'oligomères de diisocyanates et de diisocyanates monomères selon au moins l'une quelconque des revendications précédentes, dans laquelle de l'hydroxyde de tétraéthylammonium est utilisé en tant que catalyseur de trimérisation III.

11. Procédé de fabrication de compositions de trimères et/ou d'oligomères de diisocyanates et de diisocyanates monomères selon au moins l'une quelconque des revendications précédentes,
pouvant être obtenues par mise en réaction de
I. 5 à 94,999 % en poids de
A) au moins un diisocyanate aliphatique et/ou cycloaliphatique ayant un point d'ébullition inférieur à 250 °C,
et/ou
B) au moins un diisocyanate aliphatique et/ou cycloaliphatique ayant un point d'ébullition de 250 à 350 °C,
en présence de
II. 94,999 à 5 % en poids de
C) au moins un diisocyanate ayant un point d'ébullition supérieur à 350 °C choisi parmi le diisocyanate de dicyclohexylméthylène (H₁₂MDI) et/ou le diisocyanate d'octadécane,
III. en présence d'au moins un catalyseur de trimérisation en quantités de 0,001 à 5 % en poids,
la somme des quantités de I. à III. étant de 100 % en poids,
la proportion de A) monomère et/ou de B) monomère après la réaction dans le mélange, par rapport au mélange total A)+B)+C), n'étant absolument pas supérieure à 20 % en poids et, par rapport au diisocyanate A) et/ou B) utilisé, n'étant relativement pas supérieure à 40 % en poids.

12. Procédé de fabrication de compositions de trimères et/ou d'oligomères de diisocyanates et de diisocyanates monomères selon la revendication 11, dans lequel la réaction a lieu à des températures de 0 à 140 °C, de préférence de 55 à 90 °C, de manière particulièrement préférée de 65 à 75 °C.

13. Utilisation de la composition de trimères et/ou d'oligomères de diisocyanates et de diisocyanates monomères selon au moins l'une quelconque des revendications précédentes,
pouvant être obtenue par mise en réaction de
I. 5 à 94,999 % en poids de
A) au moins un diisocyanate aliphatique et/ou cycloaliphatique ayant un point d'ébullition inférieur à 250 °C,
et/ou
B) au moins un diisocyanate aliphatique et/ou cycloaliphatique ayant un point d'ébullition de 250 à 350 °C,
en présence de
II. 94,999 à 5 % en poids de
C) au moins un diisocyanate ayant un point d'ébullition supérieur à 350 °C choisi parmi le diisocyanate de dicyclohexylméthylène (H₁₂MDI) et/ou le diisocyanate d'octadécane,
III. en présence d'au moins un catalyseur de trimérisation en quantités de 0,001 à 5 % en poids,
la somme des quantités de I. à III. étant de 100 % en poids,
la proportion de A) monomère et/ou de B) monomère après la réaction dans le mélange, par rapport au mélange total A)+B)+C), n'étant absolument pas supérieure à 20 % en poids et, par rapport au diisocyanate A) et/ou B) utilisé, n'étant relativement pas supérieure à 40 % en poids,
dans des vernis, des adhésifs, des agents d'étanchéité et/ou des matières plastiques.
